# EUROPÄISCHE PATENTSCHRIFT

(11) **EP 1 402 904 B1**
(45) Veröffentlichungstag und Bekanntmachung des Hinweises auf die Patenterteilung: **29.08.2012**
(21) Anmeldenummer: 03021757.4
(22) Anmeldetag: 25.09.2003
(51) Int. Cl.: A61L 2/00, A61L 2/26, G06F 19/00, A61B 19/00, G06K 7/10, G06K 17/00, A61L 2/24

(54) **Wiederaufbereitung von medizinischen Instrumenten in einer Zentral-Sterilisationsabteilung**
Recycling of medical instruments in a central sterilisation unit
Recyclage d'instruments medicaux dans une unité de sterilisation centrale

(30) Priorität: 26.09.2002 DE 10245061
(43) Veröffentlichungstag der Anmeldung: 31.03.2004
(73) Patentinhaber: Vanguard AG, 10117 Berlin (DE)
(72) Erfinder: Segeletz, Nicos, 14109 Berlin (DE); Lieshoff, Oliver, 13597 Berlin (DE); Wünsche, Frank, 10827 Berlin (DE)
(74) Vertreter: von Puttkamer · Berngruber

(56) Entgegenhaltungen:
- EP-A- 0 630 820
- WO-A-00/57336
- WO-A-95/27252
- WO-A-99/66444
- DE-A- 3 917 876
- DE-A- 19 514 284
- DE-A- 19 614 719
- US-A- 5 374 813

## Beschreibung

Die vorliegende Erfindung betrifft ein Verfahren zur Wiederaufbereitung von medizinischen Instrumenten in einer Zentral-Sterilisationsabteilung unter Verwendung von Instrumenten-Codierungen, Scannern, meheren Computern, die mit einer zentralen Datenbank in Verbindung Stehen.

Auf Grund eins maßgeblichen Einflusses des Gesetzgebers auf das Gesundheitswesen hat sich das Umfeld desselben in den letzten Jahren drastisch verändert. Insbesondere wurde die Integration von IT-Systemen in die einzelnen Abläufe von Krankenhäusern in den letzten Jahren konsequent vorangetrieben. Die Einführung einer computerunterstützten Instrumentenverwaltung im OP und bei der Sterilisation zur Qualitätssicherung erfordert eine lückenlose Dokumentation und Rückverfolgbarkeit aller Abläufe einschließlich des kontrollierbaren und transparenten Einsatzes des gesamten Instrumentariums, dessen Handhabung und Aufbereitung.

Insbesondere erfordert eine Optimierung der Arbeitsabläufe in OP und bei der Zentralsterilisation die Erstellung von Siebplänen, die Dokumentierung und Personifizierung der Arbeitsabläufe, das Packen von Sieben, das Ersetzen von fehlenden oder zu reparierenden Instrumenten und eine zentrale Überwachung der Lagerbestände etc. Ferner sollen in einem Krankenhaus das gesamte Instrumentarium verwaltet, die Arbeitsabläufe dokumentiert und personifiziert werden.

Aus der WO 99/66444 A, DE 196 14 719 A1, DE 39 18 876 A1 und WO 95/27252 A sind beispielsweise Verfahren zur Überwachung und Steuerung der Materialflüsse, insbesondere von sterilen medizinischen Instrumenten in Krankenhäusern bekannt. Die medizinischen Instrumente sind hierbei mit einer individuellen Kenzeichnung versehen, die digital erfasst werden kann und in einer Datenbank zur Steuerung der Materialflüsse gespeichert werden kann.

In diesem Zusammenhang besteht die Aufgabe der vorliegenden Erfindung darin, ein Verfahren zur Wiederaufbereitung von medizinischen Instrumenten in Krankenhäusern zu schaffen, durch das eine die Wiederaufbereitung der Instrumente betreffende optimale Datenerfassung möglich ist.

Diese Aufgabe wird durch ein Verfahren zur Wiederaufbereitung von medizinischen Instrumenten mit den Merkmalen des Patentanspruchs 1 gelöst.

Der wesentliche Vorteil der vorliegenden Erfindung besteht darin, dass auf Grund der Zuordnung eines Codes, insbesondere eines Data-Matrix-Codes, zu den einzelnen Instrumenten in einer Zentral-Sterilisationsabteilung eines Krankenhauses eine optimale Erfassung sämtlicher die Wiederaufbereitung, die Instandhaltung, das Packen, die Auslieferung und Lagerung sowie Sterilisation der medizinischen Instrumente betreffenden Daten in einer zentralen Datenbank speicherbar und dort jederzeit abrufbar sind. Auf diese Weise wird in einem Krankenhaus eine optimale Instrumentenverwaltung ermöglicht. Insbesondere werden bei der Wiederaufbereitung sämtliche relevanten Daten ermittelt und gespeichert, die das Einliefern von gebrauchten Instrumenten in einem Sieb, das eine Sieb-Codierung besitzt, die Chargenzusamnenstellung beim Waschen der Instrumente, die Chargenfreigabe, das Siebpacken, die Chargenzusammenstellung beim Sterilisieren, die Freigabe nach dem Sterilisieren, die Sterilgutauslieferung, die Sterilgutlagerung, die Lagerverwaltung und die Sterilgut-Verwendung betreffen. Eine solche optimale Datenerfassung ist möglich, weil jedem Instrument eine spezielle Instrumenten-Codierung und jedem Sieb eine Sieb-Codierung zugeordnet sind und weil in der zentralen Sterilisationsabteilung eine Vielzahl von Computern vorgesehen sind, die die an den unterschiedlichsten Wiederaufbereitungsstationen eingelesenen Daten mit den jeweiligen Instrumentendaten verbinden und an eine zentrale Datenbank übertragen, in der diese Daten zur späteren Auswertung gespeichert werden. Auf diese Weise kann, wie dies später näher erläutert werden wird, der gesamte Einsatz sowie die gesamte Wiederaufbereitung und Instandhaltung eines Instruments während seiner gesamten Lebensdauer dokumentiert werden.

Vorteilhafte Ausgestaltungen der Erfindung gehen aus den Unteransprüchen hervor.

Im folgenden werden die Erfindung und deren Ausgestaltungen im Zusammenhang mit den Figuren näher erläutert. Es zeigen:
Figur 1 ein Blockschaltbild zur Erläuterung des erfindungsgemäßem Verfahrens zur Wiederaufbereitung von medizinischen Instrumenten und
Figur 2 ein Instrument mit einer Instrumenten-Codierung.

Im folgenden wird zunächst der an sich bekannte Ablauf der Wiederaufbereitung von medizinischen Instrumenten in einer Zentral-Sterilisationsabteilung 1 (ZSVA) näher erläutert. Die gebrauchten Instrumente werden von einer Verbrauchsstelle 2, beispielsweise einem OP-Raum oder einer Krankenhausstation in eine Waschzone 3 geliefert (Pfeil P1). Üblicherweise sind sie hierbei in einem sogenannten Sieb enthalten, das mit den verschmutzten Instrumenten in einen Waschautomaten 31 der Waschzone 3 zum Waschen der Instrumente eingebracht wird (Pfeil P2). Die Siebe mit den gewaschenen Instrumenten werden in einen Packbereich 4 gebracht (Pfeil P3), wo sie anhand von Packlisten bepackt werden (Pfeil P4). Nach dem Bepacken der Siebe werden diese in einen Sterilisator 20 (Pfeil P5) gebracht und nach der erfolgten Sterilisation (Pfeil P6) im Sterilbereich 5 entladen. Anschließend werden die sterilisierten Siebe mit den sterilisierten Instrumenten zu einem Sterilgutlager 6 gebracht (Pfeil P8).

Je nach Bedarf werden die Siebe aus dem Sterilgutlager 6 zur Verwendung zu den Verbrauchsstellen 2 gebracht, wo der Kreislauf nach der Verwendung der Instrumente erneut beginnt.

Im Packbereich 4 werden beim Bepacken der Siebe anhand der Packlisten schadhafte Instrumente zu einem Instandhaltungsbereich 7 gebracht (Pfeil P9) und bedarfsweise durch entsprechende reparierte bzw. ersetzte Instrumente, die zum Packbereich befördert werden (Pfeil P10), ersetzt. Der Instandhaltungsbereich 7 wird normalerweise von einem externen Dienstleister betrieben.

Es wird darauf hingewiesen, dass die genannten Instrumente erfindungsgemäß zur Führung, Überwachung und Protokollierung aller an den Instrumenten durchgeführten Aufbereitungsprozesse sowie zur vollständigen Abbildung der "Lebensläufe" der einzelnen Instrumente mit einer Instrument-Codierung versehen werden, bei der es sich vorzugsweise um einen Data-Matrix-Code handelt, der mit der Hilfe eines Laserkennzeichnungsautomaten auf die Instrumente aufgebracht wird. Eine solche Instrumenten-Codierung ist dauerhaft und übersteht problemlos die mechanischen, chemischen und thermischen Beanspruchungen, denen ein Instrument während seines gesamten Lebenszyklus unterworfen ist. Eine solche durch Laser aufgebrachte Instrumenten-Codierung ist vorteilhafterweise medizinisch unbedenklich und bewirkt einen Farbumschlag in der Oberfläche ohne die Einbringung zusätzlicher Pigmente. Eine mechanische Veränderung der Oberflächen, die aus Gründen der Rechts- und Patientensicherheit problematisch wäre, erfolgt dabei nicht. Die vorliegende Instrumenten-Codierung wird auf Instrumentenbereiche aufgebracht, die nicht funktionsrelevant sind und in der Regel nicht mit Patientengewebe in Kontakt kommen.

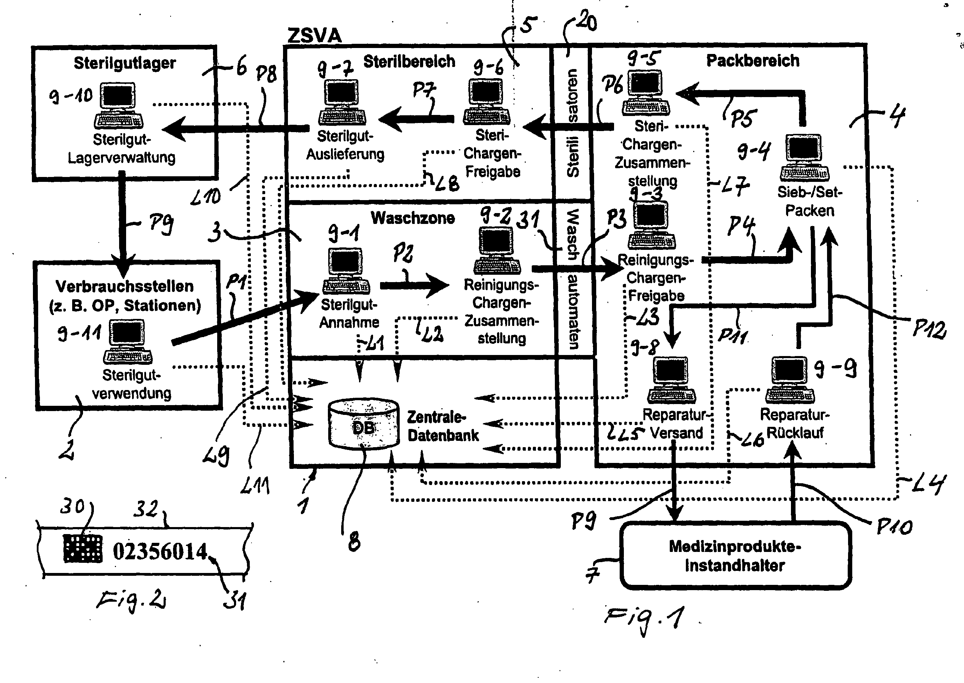

Ein Beispiel einer solchen Instrumenten-Codierung an einem Instrumentengriff 32 zeigt die Figur 2, wobei mit 30 die eigentliche Codierung, bei der es sich vorzugsweise um eine 2D-Laser-Codierung handelt, und mit 31 die in der 2D-Laser-Codierung verschlüsselte weltweit eindeutige Instrumentennummer im Klartext bezeichnet sind.

Um ein sogenanntes Instrumenten-Tracking zu ermöglichen, sind bei dem erfindungsgemäßen Verfahren neben einer zentralen Datenbank 8 an den einzelnen Zonen bzw. Bereichen der Zentral-Sterilisationsabteilung 1 Computer 9-1 bis 9-7 vorgesehen, denen jeweils ein Scanner (nicht näher bezeichnet) zum Ablesen der Instrumenten- bzw. Sieb-Codierung zugeordnet ist. Ferner weisen der Packbereich 4 weitere Computer 9-8 und 9-9, die den Reparaturversand und den Reparaturrücklauf betreffen, das Sterilgutlager 6 einen Computer 9-10 und die Verbrauchsstelle 2 einen Computer 9-11 auf. Jeder der genannten Computer 9-1 bis 9-11 ist über eine Leitung (gepunktete Linien L1 bis L11) mit der zentralen Datenbank 8 verbunden.

Die vorliegende Erfindung beruht nun darauf, dass, wie dies später näher erläutert werden wird, an jedem für die Datenerfassung wichtigen Ort der Zentral-Sterilisationsabteilung 1, zwischen dem Packbereich 4 und dem Instandhaltungsbereich 7, in dem Sterilgutlager 6 und an der Verbrauchsstelle 2 ein Computer mit Scanner zum Ablesen der Instrumenten-Codierung (Instrumenten-Tracking) bzw. der Sieb-Codierung (Sieb-Tracking) angeordnet ist.

Im einzelnen wird beim dem erfindungsgemäßen Verfahren zur Wiederaufbereitung von medizinischen Instrumenten in einer Zentral-Sterilisationsabteilung wie folgt vorgegangen. Die an einer Verbrauchsstelle 2 anfallenden, zu reinigenden und zu sterilisierenden Instrumente werden in einem Sieb abgelegt und der Waschzone 3 der Zentral-Sterilisationsabteilung 1 zugeführt (Pfeil P1). Dabei ist an dem Sieb ein Etikett mit einer Codierung befestigt, die im Computer 9-1 zur Sterilgutaufnahme in einem dem Computer 9-1 zugeordneten Lesegerät abgescannt wird. Auf diese Weise müssen nicht sämtliche in dem Sieb enthaltenen verunreinigten Instrumente an dieser Stelle, d.h. also vor dem Waschen manuell ergriffen und abgescannt werden. Die von dem Etikett des Siebs abgescannten Daten werden über die Leitung L1 an die zentrale Datenbank 8 übertragen und in dieser abgespeichert. Die abgespeicherten Daten betreffen beispielsweise: Siebnummer (für das sogenannte Sieb-Tracking) und Zeit. Am Computer 9-1 können diesen Daten weitere Daten, die beispielsweise die Annahmeperson oder die absendende Verbrauchsstelle etc. betreffen, hinzugefügt werden.

Das Sieb wird dann zur Zusammenstellung von Reinigungschargen mit anderen Sieben zusammengefasst, deren Instrumente in derselben Weise zu waschen sind. Jeweils zusammengestellte Reinigungschargen werden dann in den entsprechenden Waschautomaten 31 gewaschen. Die die Zusammenstellung von Reinigungschargen betreffenden Daten werden dann über die Leitung L2 an die zentrale Datenbank 8 übertragen. Im Packbereich werden die den Waschautomaten 31 entnommenen Siebe jeweils einer Reinigungscharge (Pfeil P3) bewertet, wobei am Computer 9-3 eine Freigabe der Reinigungschargen erfolgt. Die entsprechenden Freigabedaten werden über die Leitung L3 zur zentralen Datenbank 8 übertragen. Anschließend erfolgt im Bereich des Computers 9-4 (Zuführung entsprechend Pfeil P4) das ordnungsgemäße Packen der Siebe entsprechend einer Packliste, die am Computer 9-4 angezeigt wird. Beim Packen werden die im Waschautomaten 31 gewaschenen Instrumente im Sieb genau in der richten Reihenfolge sortiert. Hierzu zeigen die Sieblisten am Computer 9-4 genau an, wo im Sieb welches Instrument unterzubringen ist. Beim Packen wird die Instrumenten-Codierung jedes im Sieb an der richtigen Stelle abzulegenden Instruments durch einen dem Computer 9-4 zugeordneten Scanner abgelesen und über die Leitung L4 der zentralen Datenbank zugeführt. Auf diese Weise wird dokumentiert, dass das entsprechende Instrument in dem jeweiligen Sieb (Abscannen des Etiketts des Siebs) an dem richtigen Ort entsprechend der Packliste abgelegt wurde. In dem Fall, in dem ein falsches Instrument (z.B. eine zu kleine Schere) in dem Sieb abgelegt wird, erzeugt das System anhand der von dem falschen Instrument abgescannten Instrumenten-Codierung eine Fehlermeldung.

Es wird darauf hingewiesen, dass dann, wenn beim Bepacken auffällt, dass Instrumente fehlerhaft sind bzw. beschädigt sind, diese einem Instandhaltungsbereich 7 zugeführt werden (Pfeile P9 und P10), wobei sie durch einen Computer 9-8, an dessen Scanner sie abgescannt werden, erfasst werden. Die abgescannten Informationen werden über die Leitung L5 der zentralen Datenbank 8 zugeführt. Diese Daten umfassen beispielsweise Informationen darüber, wann das jeweilige Instrument z.B. mit welcher Fehlerart an den Instandhaltungsbereich 7 weitergeleitet wurde. Entsprechend werden vom Instandhaltungsbereich 7 ausgelieferte Instrumente (Pfeil P19) am Computer 9-9 erfasst, wobei ihre Instrumenten-Codierung an dem zugeordneten Scanner abgelesen wird. Die entsprechenden Daten werden über die Leitung L6 an die zentrale Datenbank geliefert. Diese Daten umfassen beispielsweise die Zeit des Rücklaufs, die Reparaturkosten, die Art der ausgeführten Reparatur etc. Danach werden die reparierten Instrumente wieder dem Bepackungsort zugeführt (Pfeil P12), um dort zum Bepacken von Sieben zur Verfügung zu stehen.

Neben dem bereits angesprochenen Sieb-Tracking, das durch die Etikettierung des Siebs mit einer Sieb-Codierung ermöglicht wird, erfolgt am Bepackungsort zusätzlich durch Abscannen der Instrumenten-Codierung (Computer 9-4) auch ein Instrumenten-Tracking. Nach dem Bepacken werden im Bereich des Computers 9-5 sogenannte Sterilchargen zusammengestellt, was bedeutet, dass die einzelnen Chargen jeweils Siebe mit Instrumenten enthalten, die dieselbe Sterilisation im Sterilisator 20 erfordern. Die Chargenzusammenstellung wird über die Leitung L7 der zentralen Datenbank 8 mitgeteilt, wobei die übermittelten Daten die für die einzelnen Chargen jeweils auszuführenden Sterilisieroperationen (z.B. Chargennummer, Sterilisationsprogramm, Temperatur etc.) bezeichnen. Nach der Sterilisierung in einem Sterilisator 20 erfolgt am Ort des Computers 9-6 (Zuführung entsprechend Pfeil P6) die Freigabe der einzelnen Chargen, wobei die entsprechenden Freigabedaten über die Leitung L8 an die zentrale Datenbank 8 übermittelt werden. Diese Daten betreffen die Bezeichnung der freigegebenen Chargen, die Zeit der Freigabe, die Mitarbeiterkennung des prüfenden Mitarbeiters etc. Am Ort des Computers 9-7 des Sterilbereichs 5 erfolgt die Sterilgut-Auslieferung (Anlieferung mit Pfeil P7), wobei die entsprechenden Daten (Sieb-Umlaufnummer, Zeitpunkt der Auslieferung, Adressat (Verbrauchsstelle/Kostenstelle) etc.) über die Leitung L9 an die zentrale Datenbank 8 geliefert werden.

Vom Ort der Sterilgut-Auslieferung werden die Siebe zum Sterilgutlager 6 (Pfeil P8) geliefert und dort werden über die Leitung L10 Daten an die zentrale Datenbank 8 geliefert, die die Anlieferung und das Ablegen im Steilgutlager 6 betreffen. Dabei können im Sterilgutlager die einzelnen Siebe modulartig bzw. chargenartig zusammengestellt werden, je nachdem, an welche bestimmten Stationen oder OPs sie zur Anwendung ausgeliefert werden (Pfeil P9).

An der Verbrauchsstelle 2 wird in einem Computer 9-10 durch Abtasten des Etiketts der einzelnen Siebe erfasst, wann, durch wen und wofür (Mitarbeiter-Kennung, Fallnummer, Patientennummer, Operateur, Operationssaal etc.) die einzelnen Siebe und die darin enthaltenen Instrumente jeweils verwendet werden. Die entsprechenden Daten werden über die Leitung L11 an die zentrale Datenbank geliefert.

Nach Anwendung der einzelnen Instrumente eines oder mehrerer Siebe werden die Instrumente in die entsprechenden Siebe eingebracht und erneut zum Ort der Sterilgutannahme in der Waschzone 9 geliefert (Pfeil P1).

## Patentansprüche

1. Verfahren zur Wiederaufbereitung von medizinischen Instrumenten in einer Zentral-Sterilisationsabteilung (1), bei dem die Instrumente mit einer Instrumenten-Codierung (30) versehen sind, die in einem Packbereich (4) der Zentral-Sterilisationsabteilung (1) beim Bepacken von Sieben ausgelesen und an eine zentrale Datenbank (8) übertragen werden, wobei:
die an Verbrauchsstellen (2) anfallenden, zu reinigenden und zu sterilisierenden Instrumente in einem Sieb abgelegt und einer Waschzone (3) der Zentral-Sterilisationsabteilung (1) zugeführt werden, wobei das Sieb eine Sieb-Codierung aufweist, die in einem ersten Computer (9-1) der Waschzone (3) zur Sterilgutannahme in einem dem ersten Computer (9-1) der Waschzone (3) zugeordneten Lesegerät abgescannt, an die zentrale Datenbank (8) übertragen und in dieser abgespeichert wird,
in dem Packbereich (4) an einem ersten Computer (9-4) des Packbereiches eine Packliste angezeigt und das ordnungsgemäße Ablegen der Instrumente in den Sieben entsprechend der Packliste ausgeführt wird, wobei die Instrumenten-Codierung jedes an der richtigen Stelle abgelegten Instruments durch einen, dem ersten Computer (9-4) des Packbereiches (4) zugeordneten Scanner abgelesen, zur zentralen Datenbank (8) übertragen und in dieser gespeichert wird,
und die in dem Packbereich (4) bepackten Siebe in einem Sterilbereich (5) der Zentral-Sterilisationsabteilung (1) sterilisiert werden, wobei die Sieb-Codierungen durch einen, einem ersten Computer (9-6) des Sterilbereiches (5) zugeordneten Scanner ausgelesen und in die zentrale Datenbank (8) übertragen werden.

2. Verfahren nach Anspruch 1, **dadurch gekennzeichnet, dass** die von dem ersten Computer (9-1) der Waschzone abgescannten und an die zentrale Datenbank (8) übertragenen Daten die Siebnummer und die Zeit fassen.

3. Verfahren nach Anspruch 1 oder 2, **dadurch gekennzeichnet, dass** in der Waschzone (3) mehrere aus Verbrauchsstellen (2) eingegangene Siebe zu Reinigungschargen zusammengefasst werden, deren Instrumente in gleicher Weise in wenigstens einem Waschautomaten (31) der Waschzone (3) zu waschen sind, wobei die die Zusammenstellung von Reinigungschargen betreffenden Daten in einem zweiten Computer (9-2) der Waschzone (3) erfasst, zur zentralen Datenbank übertragen und in dieser abgespeichert werden.

4. Verfahren nach Anspruch 3, **dadurch gekennzeichnet, dass** die dem wenigstens einen Waschautomaten (31) entnommenen Siebe jeweils einer Reinigungscharge bewertet werden, wobei an einem dritten Computer (9-3) der Waschzone (3) entsprechende Freigabedaten erzeugt, zur zentralen Datenbank (8) übertragen und in dieser abgespeichert werden.

5. Verfahren nach einem der Ansprüche 1 bis 4, **dadurch gekennzeichnet, dass** beim Bepacken im Packbereich (4) als fehlerhaft bzw. beschädigt auffallende Instrumente zu einem Instandhaltungsbereich (7) übertragen werden, wobei ihre Instrumenten-Codierung mit einem einem ersten Computer (9-8) des Instandhaltungsbereiches (7) zugeordneten Scanner abgescannt, der zentralen Datenbank (8) zugeführt und in dieser gespeichert wird.

6. Verfahren nach Anspruch 5, **dadurch gekennzeichnet, dass** der Instrumenten-Codierung Daten über die Fehlerart hinzu geführt werden.

7. Verfahren nach Anspruch 5 oder 6, **dadurch gekennzeichnet, dass** die Instrumenten-codierung von aus dem Instandhaltungsbereich (7) ausgelieferten, fehlerfreien Instrumenten an einem einem zweiten Computer (9-9) des Instandhaltungsbereiches (7) zugeordneten Scanner abgelesen, der zentralen Datenbank (8) zugeführt und in dieser abgespeichert wird.

8. Verfahren nach Anspruch 7, **dadurch gekennzeichnet, dass** der Instrumenten-Codierung Daten hinzugefügt werden, die die Zeit des Rücklaufs und/oder die Reparaturkosten und/oder die Art der ausgeführten Reparaturkosten betreffen.

9. Verfahren nach einem der Ansprüche 1 bis 8, **dadurch gekennzeichnet, dass** im Packbereich (4) nach dem Bepacken Sterilchargen zusammengestellt werden, deren Siebe jeweils Instrumente enthalten, die dieselbe Sterilisation in einem Sterilisator (20) eines Sterilbereiches (5) erfordern, wobei an einem einem zweiten Computer (9-5) des Packbereiches (4) zugeordneten Scanner die Sieb-Codierung erfasst, der zentralen Datenbank (8) zugeführt und in dieser gespeichert wird.

10. Verfahren nach Anspruch 9, **dadurch gekennzeichnet, dass** der Sieb-Codierung zur Sterilisation in dem Sterilisator (30) Daten hinzu geführt werden, die die für die einzelnen Sterilchargen jeweils auszuführenden Sterilisieroperationen bezeichnen.

11. Verfahren nach einem der Ansprüche 9 oder 10, **dadurch gekennzeichnet, dass** nach der Sterilisation in einem Sterilisator (20) des Sterilbereiches (5) die Freigabe der einzelnen Sterilchargen erfolgt, wobei an einem ersten Computer (9-6) des Sterilbereiches die Freigabedaten erzeugt, der zentralen Datenbank (8) übermittelt und in dieser gespeichert werden.

12. Verfahren nach Anspruch 11, **dadurch gekennzeichnet, dass** die Freigabedaten die Sieb-Codierung und die Zeit der Freigabe und/oder eine Mitarbeiterkennung umfassen.

13. Verfahren nach einem der Ansprüche 9 bis 12, **dadurch gekennzeichnet, dass** in dem Sterilbereich (5) die Auslieferung der Siebe an ein Sterilgutlager (6) erfolgt, wobei am Ort eines zweiten Computers (9-7) des Sterilbereiches (5) entsprechende, die Auslieferung betreffende Daten erfasst, zur zentralen Datenbank (8) übertragen und in dieser gespeichert werden.

14. Verfahren nach einem der Ansprüche 1 bis 12, **dadurch gekennzeichnet, dass** an Verbrauchsstellen (2) an einem einem Computer (9-11) der Verbrauchsstellen (2) zugeordneten Scanner die Sieb-Codierung der einzelnen Siebe erfasst wird, wobei Daten, die die Anwendung der in den Sieben enthaltenen Instrumente betreffen hinzugefügt, zur zentralen Datenbank (8) übertragen und in dieser gespeichert werden.

15. Verfahren nach Anspruch 14, **dadurch gekennzeichnet, dass** die Daten eine Mitarbeiter-Kennung und/oder eine Fallnummer und/oder eine Patientennummer und/oder einen Operateur und/oder einen Operationssaal betreffen.

## Claims

1. A method for recycling of medical instruments in a central sterilisation unit (1), in which the instruments are provided with an instrument coding (30), which is read out in a packing area (4) of the central sterilisation unit (1) during the loading of the sieves and transferred to a central data base (8), wherein:
the instruments to be cleaned and sterilised accumulating at the places of consumption (2) are deposited in a sieve and supplied to a washing zone (3) of the central sterilisation unit (1), wherein the sieve has a sieve coding, which is scanned on a first computer (9-1) of the washing zone (3) for receiving the sterile goods in a reading device assigned to the first computer (9-1) of the washing zone (3), transferred to the central data base (8) and stored therein,
a packing list is displayed in the packing area (4) on a first computer (9-4) of the packing area and the instruments are properly deposited in the sieves according to the packing list, wherein the instrument coding of each instrument deposited in the appropriate place is read by a scanner assigned to the first computer (9-4) of the packing area (4), transferred to the central data base (8) and stored therein,
and the sieves loaded in the packing area (4) are sterilised in a sterile area (5) of the central sterilisation unit (1), wherein the sieve codings are read out by a scanner assigned to a first computer (9-6) of the sterile area (5) and transferred to the data base (8).

2. The method according to claim 1, **characterized in that** the data scanned by the first computer (9-1) of the washing zone and transferred to the central data base (8) concern the sieve number and time.

3. The method according to claim 1 or 2, **characterized in that** in the washing zone (3) a plurality of sieves received from places of consumption (2) are combined into cleaning batches, the instruments of which have to be washed in the same way in at least one washing machine (31) of the washing zone (3), wherein the data relating to the combination of cleaning batches are captured on a second computer (9-2) of the washing zone (3), transferred to the central data base and stored therein.

4. The method according to claim 3, **characterized in that** the sieves of each cleaning batch removed from the at least one washing machine (31) are evaluated, wherein relevant release data are generated on a third computer (9-3) of the washing zone (3), transferred to the central data base (8) and stored therein.

5. The method according to claims 1 to 4, **characterized in that** instruments which are identified as defective and/or damaged during the loading of the sieves in the packing area (4) are conveyed to a maintenance area (7), wherein their instrument coding is scanned with a scanner assigned to a first computer (9-8) of the maintenance area (7), fed into the central data base (8) and stored therein.

6. The method according to claim 5, **characterized in that** data on the type of defect are added to the instrument coding.

7. The method according to claim 5 or 6, **characterized in that** the instrument coding of instruments which are free from defects and supplied from the maintenance area (7) is read out by a scanner assigned to a second computer (9-9) of the maintenance area (7), fed into the central data base (8) and stored therein.

8. The method according to claim 7, **characterized in that** data relating to the return time and/or repair costs and/or the type of cost of the repair carried out are added to the instrument coding.

9. The method according to one of claims 1 to 8, **characterized in that** after packing sterile batches are arranged in the packing area (4), the sieves of which each contain instruments requiring the same sterilisation in a steriliser (20) of a sterilisation area (5), wherein the sieve coding is captured by a scanner assigned to a second computer (9-5) of the packing area (4), fed into the central data base (8) and stored therein.

10. The method according to claim 9, **characterized in that** data indicating the sterilisation operations to be carried out for the individual sterile batches are added to the sieve coding for sterilisation in the steriliser (20).

11. The method according to one of claims 9 or 10, **characterized in that** the individual sterile batches are released after sterilisation in a steriliser (20) of the sterile area (5), wherein the release data are generated on a first computer (9-6) of the sterile area (5), transferred to the central data base and stored therein.

12. The method according to claim 11, **characterized in that** the release data comprise the sieve coding and release time and/or employee identification.

13. The method according to one of claims 9 to 12, **characterized in that** the sieves are supplied from the sterile area (5) to a sterile goods store (6), wherein the relevant data relating to the supply are captured at the place of a second computer (9-7) of the sterile area (5), transferred to the central data base (8) and stored therein.

14. The method according to one of claims 1 to 12, **characterized in that** the sieve coding of the individual sieves is captured at places of consumption (2) by a scanner assigned to a computer (9-11) of the places of consumption (2), wherein data relating to the use of the instruments contained in the sieves are added, transferred to the central data base (8) and stored therein.

15. The method according to claim 14, **characterized in that** the data relate to employee identification and/or a case number and/or a patient number and/or a surgeon and/or a surgery.

## Revendications

1. Procédé de recyclage d'instruments médicaux dans une unité de stérilisation centrale (1), dans lequel les instruments sont pourvus de codages d'instruments (30) qui sont lus dans une zone d'emballage (4) de l'unité de stérilisation centrale (1) lors de l'emballage de tamis et transmis à une banque de données centrale (8), dans lequel
les instruments à nettoyer et à stériliser, se présentant aux postes de consommation (2), sont déposés dans un tamis et amenés à une zone de lavage (3) de l'unité de stérilisation centrale (1), le tamis présentant un codage de tamis qui est scanné dans un premier ordinateur (9-1) de la zone de lavage (3) pour réceptionner le produit stérilisé dans un appareil de lecture associé au premier ordinateur (9-1) de la zone de lavage (3), transmis à la banque de donnée centrale (8) et mémorisé dans celle-ci,
dans la zone d'emballage (4), une liste d'emballage est affichée sur un premier ordinateur (9-4) de la zone d'emballage, et le dépôt réglementaire des instruments dans les tamis est effectué selon la liste d'emballage, le codage d'instrument de chaque instrument déposé au bon endroit étant lu par un scanner associé au premier ordinateur (9-4) de la zone d'emballage (4), transmis à la banque de données centrale (8) et mémorisé dans celle-ci,
et les tamis emballés dans la zone d'emballage (4) sont stérilisés dans une zone stérile (5) de l'unité de stérilisation centrale (1), les codages de tamis étant lus par un scanner associé à un premier ordinateur (9-6) de la zone stérile (5) et transmis à la banque de donnée centrale (8).

2. Procédé selon la revendication 1, **caractérisé en ce que** les données scannées par le premier ordinateur (9-1) de la zone de lavage et transmises à la banque de données centrale (8) saisissent le numéro de tamis et le temps.

3. Procédé selon la revendication 1 ou 2, **caractérisé en ce que** dans la zone de lavage (3), plusieurs tamis en provenance de postes de consommation (2) sont réunis en charges de nettoyage dont les instruments doivent être lavés de la même façon dans au moins une machine à laver (31) de la zone de lavage (3), les données concernant la composition de charges de nettoyage étant saisies dans un deuxième ordinateur (9-2) de la zone de lavage (3), transmises à la banque de données centrale et mémorisées dans celle-ci.

4. Procédé selon la revendication 3, **caractérisé en ce que** les tamis d'une charge de nettoyage respective extraits de ladite au moins une machine à laver (31) sont évalués, des données de validation correspondant à la zone de lavage (3) étant engendrées sur un troisième ordinateur (9-3), transmises à la banque de données centrale (8) et mémorisées dans celle-ci.

5. Procédé selon l'une des revendications 1 à 4, **caractérisé en ce que** des instruments qui sont remarqués comme étant défectueux ou détériorés lors de l'emballage dans la zone d'emballage (4) sont transmis à une zone de maintenance (7), leur codage d'instruments étant scanné avec un scanner associé à un premier ordinateur (9-8) de la zone de maintenance (7), amené à la banque de données (8) centrale et mémorisé dans celle-ci.

6. Procédé selon la revendication 5, **caractérisé en ce qu'**au codage d'instruments sont ajoutées des données sur le type d'erreur.

7. Procédé selon la revendication 5 ou 6, **caractérisé en ce que** le codage d'instruments, d'instruments exempts d'erreurs et délivrés depuis la zone de maintenance (7), est lu par un scanner associé à un deuxième ordinateur (9-9) de la banque de données centrale (8) et mémorisé dans celle-ci.

8. Procédé selon la revendication 7, **caractérisé en ce qu'**au codage d'instruments sont ajoutées des données qui concernent le temps de retour et/ou les coûts de réparation et/ou le type des coûts de réparations effectuées.

9. Procédé selon l'une des revendications 1 à 8, **caractérisé en ce que** dans la zone d'emballage (4), après l'emballage, sont regroupées des charges stériles dont les tamis contiennent chacun des instruments qui requièrent la même stérilisation dans un stérilisateur (20) d'une zone stérile (5), et au niveau d'un scanner associé au deuxième ordinateur (9-5) de la zone d'emballage, le codage de tamis étant saisi, amené à la banque de données (8) centrale et mémorisé dans celle-ci.

10. Procédé selon la revendication 9, **caractérisé en ce qu'**au codage de tamis pour la stérilisation dans le stérilisateur (20) sont ajoutées des données qui indiquent les opérations de stérilisation à exécuter respectivement.

11. Procédé selon l'une des revendications 9 ou 10, **caractérisé en ce qu'**après la stérilisation dans un stérilisateur (20) de la zone stérile (5) a lieu la validation des charges stériles individuelles, les données de validation étant engendrées sur un premier ordinateur (9-6) de la zone stérile, transmises à la banque de données (8) centrale et mémorisées dans celle-ci.

12. Procédé selon la revendication 11, **caractérisé en ce que** les données de validation comprennent le codage de tamis et le temps de la validation et/ou un identifiant de personnel.

13. Procédé selon l'une des revendications 9 à 12, **caractérisé en ce que** dans la zone stérile (5) a lieu la livraison des tamis à un dépôt de produits stériles (6), et à l'emplacement d'un deuxième ordinateur (9-7) de la zone stérile (5), des données correspondantes, concernant la livraison, sont saisies, transmises à la banque de données (8) centrale et mémorisées dans celle-ci.

14. Procédé selon l'une des revendications 1 à 12, **caractérisé en ce que** le codage de tamis des tamis individuels est saisi à des postes de consommation (2) au niveau d'un scanner associé à un ordinateur (9-11) des postes de consommation, des données qui concernent l'utilisation des instruments contenus dans les tamis étant ajoutées, transmises à la banque de données (8) centrale et mémorisées dans celle-ci.

15. Procédé selon la revendication 14, **caractérisé en ce que** les données concernent un identifiant de personnel et/ou un numéro de dossier et/ou un numéro de patient et/ou un opérateur et/ou une salle d'opération.
